# EUROPEAN PATENT APPLICATION

(11) **EP 4 344 556 A1**
(43) Date of publication of application: **03.04.2024**
(21) Application number: 22833682.2
(22) Date of filing: 30.06.2022
(51) Int. Cl.: A23L 33/10, A23L 33/105, A23P 10/20, A61K 8/49, A61K 8/9789, A61Q 5/00, A61Q 7/00, A61K 31/353, A61P 17/14

(54) **COMPOSITION COMPRISING HYDRANGENOL AS ACTIVE INGREDIENT FOR IMPROVING HAIR OR SCALP CONDITION**

(30) Priority: 30.06.2021 KR 20210085806
(71) Applicant: Cosmax Bio Co., Ltd., Jecheon-si, Chungcheongbuk-do 27159 (KR)
(72) Inventor: SHIN, Yu Kyong, Yongin-si Gyeonggi-do 16874 (KR); KIM, Hyoun Jea, Seoul 04345 (KR); AHN, Hye Shin, Bucheon-si Gyeonggi-do 14550 (KR); NA, Chang Seon, Seongnam-si Gyeonggi-do 13498 (KR)
(74) Representative: Vossius & Partner Patentanwälte Rechtsanwälte mbB
(86) International application number: PCT/KR2022/009464
(87) International publication number: WO 2023/277629

(57) **Abstract**

Provided is a composition for improving a hair or scalp condition including hydrangenol as an active ingredient. The composition including hydrangenol as an active ingredient inhibits the activity of 5α-reductase, which is a hair loss mediating factor, promotes growth of dermal papilla cells, alleviates roughness of hair cuticles, and increases the tensile strength of hair. Accordingly, the composition can be effectively used to improve the condition of hair or scalp.

## Description

### Technical Field

The present disclosure relates to a composition for improving a hair or scalp condition including hydrangenol as an active ingredient.

### Background Art

Hair is an appendage of the skin and is divided into the hair shaft, which is the outer part of the skin, and the hair root, which is surrounded by hair follicles inside the scalp. The hair corresponding to the hair shaft consists of three parts: the cuticle, the cortex, and the medulla. The cuticle is a layer of dead skin cells formed to protect the inside of the hair from external stimuli and determines the hair's wettability and gloss. The cortex and the medulla maintain the shape of the hair and are filled with fibers made of a protein called keratin. Hair is formed from the hair follicles of the hair root, and there are about 100,000 to 150,000 hair follicles on the scalp. The hair follicle is a three-layer cylindrical shape surrounding the hair in the center, and includes the dermal sheath, outer hair root sheath, and inner hair root sheath, aligned from the outermost layer. The root part of the hair follicle is called the hair bulb, in which hair is created and grows. Under the hair root, lies a structure called the dermal papilla, which is a bulb-shaped convex, papilla-like protrusion. The dermal papilla is the center that regulates hair growth, and comes from the dermal cell layer and has many capillaries and autonomic nerves. Accordingly, various nutrients, protein synthase, hormones, and oxygen, necessary for hair growth, are provided thereto. The dermal papilla is also the area where male hormones act in the case of male pattern hair loss. The area where cell division occurs under the control of the dermal papilla is called hair matrix and undergoes continuous cell division and proliferation. Sebaceous glands exist in the diagonal direction of the dermal papilla, and provide shine and oil to the hair.

The hair cycle refers to the cycle of generation and shedding of each hair. The hair cycle repeats a certain cycle of generation , growth, hair loss, and generation. Usually, the cycle is classified into an Anagen phase, a Catagen phase, and Telogen phase. The Anagen phase is a period when hair matrix cells are divided to create new hair bulbs, and the hair begins to grow within the hair follicles. This phase is the period when hair grows due to the activity of hair follicles and cell division and lasts for about 2 years to 6 years and accounts for 85% of all hair. The Catagen phase is the period when hair matrix cells stop the division thereof and begin preparing for hair loss. The hair root moves upward, the hair dermal papilla is detached from the hair bulb, and the hair bulb becomes thinner and longer like a club while contracting the tissue. The Catagen phase lasts about 2 weeks to about 3 weeks and accounts for 5% of all hair. The Telogen phase is the period in which the dermal papilla and hair fall out and the dermal papilla becomes round, and the cellular activity of the hair follicle completely disappears. Hair falls out due to being pushed by growing hair or due to mechanical action such as combing. This corresponds to 10% of all hair.

Hair loss occurs when androgens or reactive oxygen species stimulate the dermal papilla cells. This occurs when dermal papilla cells secrete substances that destroy hair follicles or inhibit the secretion of growth factors that grow hair. Hair loss also occurs even when there is an overproduction of sebum by the sebaceous glands, which causes inflammation of the dermal papilla, making the supply of nutrients inevitable. Stress is also one of the factors that cause hair loss. Corticotropin releasing hormone (CRH), which is a hormone involved in the stress response, changes the expression of cytokines in dermal papilla cells, leading to early degeneration of hair follicles and inhibiting hair growth, causing hair loss.

The main factor of male pattern hair loss is the influence of abnormal androgens. Androgens are a general term for hormones that affect the growth and development of the male reproductive system. Testosterone, one of the androgens, is converted into dihydrotestosterone, an active male hormone, by an enzyme called 5α-reductase. When activated dihydrotestosterone binds to the androgen receptor on the dermal papilla in the frontal and parietal regions, a cell destruction signal is transmitted to the DNA of hair matrix cells. When cell apoptosis factors DKK-1 and TGF-β1 are produced by this signal, hair matrix cells are destroyed and converted into a catagen phase, causing hair loss. Relatively, women only have half the amount of 5-alpha-reductase compared to men, so that the frequency of hair loss due to the same mechanism is significantly lower than that of men.

Hair damage is a phenomenon in which a space between cuticle layers is created and thus cuticle layers are lifted or destroyed, reducing the number of layers, or the bonding strength of the membrane complex is reduced, causing the cuticle layers to separate from each other. When hair is initially damaged, cuticles break or peel off, causing the hair to lose its shine. Afterwards, as the damage progresses further, cuticles almost disappears, the outer protective layer disappears, the protein inside the hair is lost, and elasticity is decreased. In addition to physiological hair loss caused by hair loss, hair damage caused by external factors can be divided into physical and chemical causes. Physical and chemical influences increase the degree of damage caused by each other. Examples of physical damage include technical stimulation such as brushing or back hair, or heat, which mainly results in morphological changes. Chemical effects include qualitative damage to hair components caused by waving agents or dyes.

Hydrangenol has a molecular weight of 256.25 g/mol and the IUPAC name is 8-hydroxy-3- (4-hydroxyphenyl)-3,4-dihydroisochromen-1-one. Additionally, derivatives of hydrangenol include (-)-hydrangenol 4'-O-glucoside, (+)-hydrangenol 4'-O-glucoside. Hydrangenol has been reported to have skin whitening (Japanese public patent JP-0007546) and anti-inflammatory effects (International immunopharmacology v.35, pp. 61 - 69 , 2016, 1567-5769).

However, the use of hydrangenol in the prevention, improvement, or treatment of hair and scalp conditions is not known and no mechanisms of action thereof have been studied. Therefore, the inventors of the present application conducted a direct study on the efficacy of hydrangenol in preventing, improving, and treating hair and scalp health.

### Detailed Description of the disclosure

### Technical Problem

One aspect is to provide a health functional food composition for improving a hair or scalp condition including hydrangenol represented by Formula 1 or a foodologically acceptable salt thereof as an active ingredient:

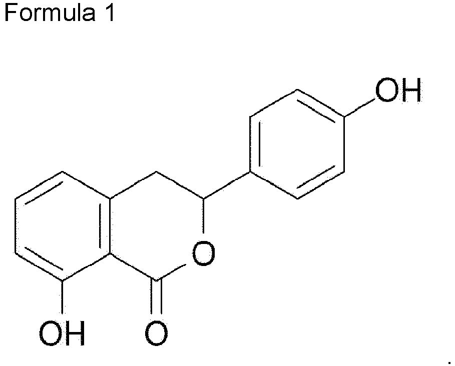

One aspect is to provide a cosmetic composition for improving a hair or scalp condition including hydrangenol represented by Formula 1 or a cosmetically acceptable salt thereof as an active ingredient.

Another aspect is to provide a pharmaceutical composition for preventing or treating hair loss including hydrangenol represented by Formula 1 or a pharmaceutically acceptable salt thereof as an active ingredient.

Another aspect is to provide an external skin preparation for improving a hair or scalp condition including hydrangenol represented by Formula 1 or a pharmaceutically acceptable salt thereof as an active ingredient.

Another aspect provides a method of preventing, alleviating, or treating hair loss including administering an effective amount of hydrangenol represented by Formula 1 or a pharmaceutically acceptable salt thereof to a subject in need thereof.

Another aspect is to provide use of hydrangenol represented by Formula 1 or a pharmaceutically acceptable salt thereof in the manufacture of a composition for improving a hair or scalp condition, or preventing, alleviating or treating hair loss.

### Technical Solution to Problem

One aspect is to provide a health functional food composition for improving a hair or scalp condition including hydrangenol represented by Formula 1 or a foodologically acceptable salt thereof as an active ingredient:

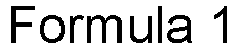

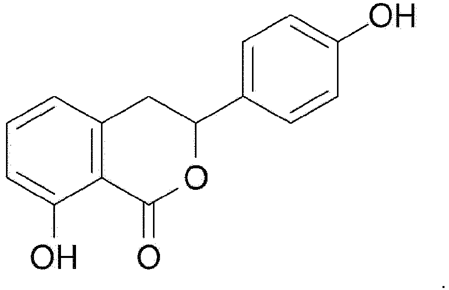

The hydrangenol can be purchased and used on the market, can be synthesized according to methods of the related art. In some embodiments, hydrangenol can be separated and purified from a natural product and used. In an embodiment, hydrangenol may be isolated from *Hydrangea serrata* extract.

The *Hydrangea serrata* extract may be an extract of the entire above-ground part of the tree, a portion thereof, or materials derived therefrom, which is obtained using a solvent. The portion may be the stem, root, leaf, flower, petal (seed), pulp, fruit peel or fruit of the tree, and may be a leaf. The whole tree, a portion thereof, or a material derived therefrom used for extraction may be ground, shredded, or suitably dried.

The *Hydrangea serrata* extract may be obtained using extraction methods of the related art which are used to extract natural plants, such as hot water extraction, solvent extraction, distillation extraction, or supercritical extraction. In some embodiments, the *Hydrangea serrata* extract may be obtained by extraction using water, an organic solvent, or a mixed solvent thereof. The organic solvent may be one or more selected from the group consisting of alcohols having 1 to 4 carbon atoms, such as ethanol, methanol, isopropanol, and butanol. In some embodiments, if necessary, after the extraction, filtration and/or concentration and/or freeze-drying methods known in the art may be additionally used.

The hydrangenol may be a fraction of the *Hydrangea serrata* extract. The fraction refers to a material which is separated from the extract and includes a specific component. The hydrangenol may be separated and purified using column chromatography. The chromatography may include silica gel column chromatography, HP-20 column chromatography, RP-18 column chromatography, and LH-20 column chromatography, high-performance liquid chromatography, or a combination thereof.

In an embodiment, the improving the hair or scalp condition may include preventing or alleviating hair damage, promoting hair growth, or suppressing, preventing or improving hair loss. The inventors of the present application confirmed that the hydrangenol inhibits 5α-reductase and promotes the proliferation of dermal papilla cells, and thus has the effect of promoting hair growth and inhibiting, preventing or alleviating hair loss. In addition, it was confirmed that the hydrangenol increases the tensile strength of the hair and alleviates roughness of hair cuticles, and thus, the hydrangenol has an effect in preventing or improving hair damage.

In an embodiment, the hydrangenol may be included in an amount of 0.0001 wt% to 99.0 wt%, for example, 0.01 wt% to 60 wt%, 0.01 wt% to 40 wt%, 0.01 wt% to 30 wt%, 0.01 wt% to 20 wt%, 0.01 wt% to 10 wt%, 0.01 wt% to 5 wt%, 0.05 wt% to 60 wt%, 0.05 wt% to 40 wt%, 0.05 wt% to 30 wt%, 0.05 wt% to 20 wt%, 0.05 wt% to 10 wt%, 0.05 wt% to 5 wt%, 0.1 wt% to 60 wt%, 0.1 wt% to 40 wt%, 0.1 wt% to 30 wt%, 0.1 wt% to 20 wt%, 0.1 wt% to 10 wt%, 0.1 wt% to 5 wt%, 5 to 20 wt%, 5 to 18 wt%, 6 to 15 wt%, 8 to 15 wt%, or 7 to 10 wt%, based on the total weight of the composition. However, in the case of long-term intake for the purpose of health and hygiene or health control, the amount may be smaller than the lower limits of these ranges, and since there is no problem in terms of safety, the active ingredient may be used in amounts greater than the upper limits of these ranges.

The hydrangenol may be added as is or used with other foods or food ingredients, and may be used appropriately according to conventional methods. Additionally, the composition may further include *Hydrangea serrata* extract.

The term "acceptable" as used herein refers to non-toxic properties to cells or humans exposed to the composition.

The term "acceptable salt" as used herein refers to a salt prepared using a specific compound according to one aspect and a relatively non-toxic acid or base. When the compound contain a relatively acidic functional group, base addition salts may be obtained by bringing the neutral form of these compounds to be in contact with a sufficient amount of base in pure solution or a suitable inactive solvent. Pharmaceutically acceptable base addition salts include salts of sodium, potassium, calcium, ammonium, organic amines, or magnesium, or salts similar thereto. When the compound contain a relatively basic functional group, acid addition salts may be obtained by bringing the neutral form of these compounds to be in contact with a sufficient amount of acid in pure solution or a suitable inactive solvent. Pharmaceutically acceptable acid addition salts include salts of inorganic acids such as hydrochloric acid, hydrobromic acid, nitric acid, carbonic acid, hydrogen carbonate ion, phosphoric acid, monohydrogen phosphate ion, dihydrogen phosphate ion, sulfuric acid, hydrogen sulfate ion, hydroiodic acid, or phosphorous acid, and salts of organic acids such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-tolylsulfonic acid, citric acid, tartaric acid, and methanesulfonic acid, and further include salts of amino acids (e.g., arginine, etc.) and salts of organic acids, such as glucuronic acid.

The acceptable salts may be synthesized by chemical methods of the related art from parent compounds including an acidic or basic moiety. Generally, these salts may be prepared by reacting the free acid or base form of these compounds with a stoichiometrically appropriate amount of base or acid in water or an organic solvent or a mixture of these two different components. In some embodiments, non-aqueous media such as ether, ethyl acetate, ethanol, isopropanol, or acetonitrile may be used.

The "health functional food" refers to a food manufactured or processed using specific ingredients as raw materials or a food manufactured or processed by methods such as extraction, concentration, purification, mixing, etc. of specific ingredients contained in food raw materials, for the purpose of health supplementation, and may refer to a food designed and processed to fully exert bioregulatory functions on the living body, such as defense, regulation of biological rhythm, prevention and recovery of disease, etc.

There are no special restrictions on the types of foods above. Examples of foods, to which these materials may be added, include formulations selected from the group consisting of powder, granules, tablets, capsules, pills, gels, jellies, suspensions, emulsions, syrups, tea bags, leached teas, and health drinks. The food as used herein may include all health foods in the conventional sense.

Like drinks of the related art, the health drink composition according to the present disclosure may include various flavoring agents or natural carbohydrates as additional ingredients. The natural carbohydrates may include monosaccharides such as glucose and fructose, disaccharides such as maltose and sucrose, natural sweeteners such as dextrin and cyclodextrin, or synthetic sweeteners such as saccharin and aspartame. The proportion of natural carbohydrates may be about 0.01 g to about 10 g, or about 0.01 g to about 0.1 g, per 100 ml of the composition of the present disclosure.

The health functional food may include food supplements that are foodologically acceptable, and may include an appropriate carrier commonly used in the manufacture of health functional foods.

In some embodiments, the composition of the present disclosure may further include various nutrients, vitamins, electrolytes, flavors, colorants, pectic acid and salts thereof, alginic acid and salts thereof, organic acids, protective colloidal thickeners, pH adjusters, stabilizers, preservatives, glycerin, alcohol, carbonating agents used in carbonated drinks, etc. Additionally, the composition of the present disclosure may include natural fruit juice, and fruit pulp for the production of fruit juice beverages and vegetable beverages. These ingredients may be used independently or in combination. The ratio of these additives is not very important, may be selected in the range of 0.01 parts by weight to 0.1 parts by weight per 100 parts by weight of the composition of the present disclosure.

One aspect is to provide a cosmetic composition for improving a hair or scalp condition including hydrangenol represented by Formula 1 or a cosmetically acceptable salt thereof as an active ingredient.

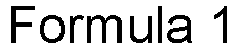

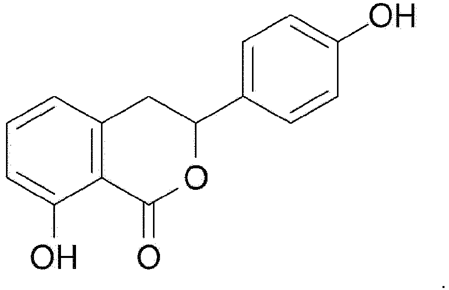

In an embodiment, hydrangenol may be isolated from *Hydrangea serrata* extract.

In an embodiment, the improving the hair or scalp condition may include preventing or alleviating hair damage, promoting hair growth, or suppressing, preventing or improving hair loss.

The cosmetic composition may be prepared in various forms, for example, a solution, a suspension, an emulsion, paste, gel, cream, lotion, shampoo, surfactant-containing cleansing, tonic, scaling, etc. For example, the cosmetic composition may be prepared in the form of hair tonic, hair conditioner, hair essence, hair lotion, hair nutrition lotion, hair shampoo, hair rinse, hair treatment, hair cream, hair nutrition cream, hair moisture cream, hair massage cream, hair wax, hair aerosol, hair pack, hair nutrition pack, hair soap, hair cleansing foam, hair oil, hair dryer, hair preservation treatment, hair dye, hair waving agent, hair bleach, hair gel, hair glaze, hair dressinger, hair lacquer, hair moisturizer, hair mousse, or hair spray. Compositions in these formulations may be prepared according to methods that are conventional in the art. The mixing amount of additional ingredients such as the moisturizer may be easily selected by a person skilled in the art within such a range that does not impair the purpose and effect of the present disclosure.

In addition to the active ingredients disclosed herein, the cosmetic composition may further include functional additives and ingredients included in general cosmetic compositions. In some embodiments, purified water, thickeners, preservatives, stabilizers, solubilizers, surfactants, carriers, fragrance or a combination thereof may be further included. The functional additive may include ingredients selected from the group consisting of water-soluble vitamins, oil-soluble vitamins, polymer peptides, polymer polysaccharides, sphingolipids, and seaweed extract. Examples of the carrier include alcohol, oil, surfactant, fatty acid, silicone oil, a wetting agent, a humectant, a viscosity modifier, an emulsion, a stabilizer, an ultraviolet scattering agent, an ultraviolet absorber, a coloring agent, fragrance, etc. Compounds/compositions that can be used as alcohol, oil, surfactant, fatty acid, silicone oil, a wetting agent, a humectant, a viscosity modifier, an emulsion, a stabilizer, an ultraviolet scattering agent, an ultraviolet absorber, a coloring agent, or fragrance are already known in the art. Therefore, a person skilled in the art can select and use the appropriate material/composition. In addition, the cosmetic composition may further include, according to purpose, sunscreen, antioxidants (butylhydroxyanisole, propyl gallate, elisorbic acid, tocopheryl acetate, butylated hydroxytoluene, etc.), preservatives (methylparaben, butylparaben, propylparaben, phenoxyethanol, imidazolidinyl urea, chlorphenesin, etc.), colorants, pH adjusters (triethanolamine, citric acid, citric acid, sodium citrate, malic acid, sodium malate, phmalic acid, sodium phmalate, succinic acid , sodium succinate, sodium hydroxide, sodium monohydrogen phosphate, etc.), humectants (glycerin, sorbitol, propylene glycol, butylene glycol, hexylene glycol, diglycerin, betaine, glycereth-26, methyl glueseth-20, etc.), and lubricants.

Additionally, regarding each formulation type of cosmetic composition, appropriate ingredients may be selected and mixed depending on the formulation or purpose of use of the cosmetic. Since the mixing ingredients and methods may be in accordance with conventional techniques, detailed descriptions thereof are omitted in this specification.

In some embodiments, the cosmetic composition may be used by applying or dispersing directly to the hair or the scalp. Hair to which the composition of the present disclosure is applied includes hair roots and hair follicles of the head, hair, eyelashes, outer eyebrows, beard, armpits, pubic hair, and all parts of the body with hair roots and hair follicles.

Another aspect is to provide a pharmaceutical composition for preventing or treating hair loss including hydrangenol represented by Formula 1 or a pharmaceutically acceptable salt thereof as an active ingredient.

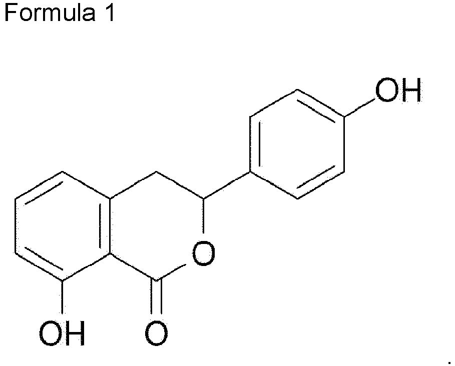

Another aspect provides a method of preventing, alleviating, or treating hair loss including administering an effective amount of hydrangenol represented by Formula 1 or a pharmaceutically acceptable salt thereof to a subject in need thereof.

Another aspect is to provide use of hydrangenol represented by Formula 1 or a pharmaceutically acceptable salt thereof in the manufacture of a composition for improving a hair or scalp condition, or preventing, alleviating or treating hair loss.

The term "pharmaceutical composition" may refer to a molecule or compound that imparts some beneficial effects upon administration to a subject. Beneficial effects include: enabling diagnostic decisions; alleviating of a disease, a symptom, a disorder, or a condition; reducing or preventing the onset of a disease, a symptom, a disorder, or a condition; and responding to a disease, a symptom, a disorder, or a condition. According to the present disclosure, the pharmaceutical composition may provide a beneficial effect on hair loss.

The term "prevention" refers to partially or completely delaying or preventing the onset or recurrence of a disease, a disorder, or concomitant symptoms thereof, preventing the acquisition or re-acquisition of a disease or a disorder, or reducing the risk of acquisition of a disease or a disorder. For example, the prevention refers to all actions that suppress or delay the occurrence of hair loss symptoms by administering the composition according to the present disclosure. The term "treatment" includes inhibiting, alleviating or eliminating the development of a disease, such as hair loss.

The term "alleviation" may refer to any action that alleviates a condition or at least reduces the severity of treatment-related parameters, such as hair loss symptoms.

The pharmaceutical composition may be administered parenterally during clinical administration and may be used in the form of a general pharmaceutical preparation. Parenteral administration may refer to administration through routes of administration other than oral, such as rectal, intravenous, peritoneal, muscular, arterial, transdermal, nasal, inhalation, ocular, and subcutaneous. When the pharmaceutical composition of the present disclosure is used as a medicine, the pharmaceutical composition may additionally contain one or more active ingredients that exhibit the same or similar functions.

Types of pharmaceutically active ingredients that can deliver the active ingredient into the subject include anticancer agents, contrast agents (dyes), hormones, antihormones, vitamins, calcium agents, mineral preparations, saccharides, organic acid preparations, protein amino acid preparations, detoxifiers, enzyme preparations, metabolic preparations, diabetic preparations, tissue revitalization preparations, chlorophyll preparations, dye preparations, oncological drugs, tumor treatment drugs, radiopharmaceuticals, tissue cell diagnostic agents, tissue cell therapeutic agents, antibiotic preparations, antivirals, combination antibiotic preparations, chemotherapeutic agents, vaccines, toxins, toxoids, antitoxins, leptospira serum, blood preparations, biological preparations, analgesics, immunogenic molecules, antihistamines, allergy medications, non-specific immunogenic agents, anesthetics, stimulants, psychotropic agents, small molecule compounds, nucleic acids, aptamers, antisense nucleic acids, oligonucleotides, peptides, siRNA, and micro RNA.

The pharmaceutical composition may be prepared by additionally including one or more pharmaceutically acceptable carriers. Pharmaceutically acceptable carriers may be saline solution, sterile water, a Ringer's solution, a buffered saline solution, a dextrose solution, a maltodextrin solution, glycerol, ethanol, and a mixture of one or more of these ingredients, and if necessary, other common additives such as antioxidants, buffer solutions, bacteriostatic agents, or the like may be added. In addition, diluents, dispersants, surfactants, binders, and lubricants may be additionally added to formulate injectable formulations such as aqueous solutions, suspensions, emulsions, etc., pills, capsules, granules, or tablets. Furthermore, by using an appropriate method in the art, preparation may be appropriately performed according to each disease or ingredient.

When the pharmaceutical composition is formulated, diluents or excipients, such as commonly used fillers, extenders, binders, wetting agents, disintegrants, and surfactants, may be used. Preparations for parenteral administration include sterilized aqueous solutions, non-aqueous solutions, suspensions, emulsions, freeze-dried preparations, and suppositories. Non-aqueous solvents and suspensions may include propylene glycol, polyethylene glycol, vegetable oil such as olive oil, and injectable ester such as ethyl oleate. As a base for suppositories, Witepsol, Macrogol, Tween 61, cacao, Liurinji, glycerogeratin, etc. may be used.

To increase stability or absorption, the pharmaceutical composition may include, as agents, carbohydrates such as glucose, sucrose, or dextran, antioxidants such as ascorbic acid or glutathione, chelating agents, small-molecule proteins, or other stabilizers.

Another aspect provides a method of treating hair loss including administering the pharmaceutical composition to a subject.

The term "administration" refers to providing a pharmaceutical composition to a subject by any suitable method.

The pharmaceutically effective amount and effective dosage of the pharmaceutical composition may vary depending on the preparation method, administration method, administration time, and/or administration route of the pharmaceutical composition. In addition, the pharmaceutically effective amount and effective dosage of the pharmaceutical composition may vary depending on several factors and well known analogues in the medical field, for example, the type and degree of response to be achieved by administration of the pharmaceutical composition, the type of subject to be administered, age, body weight, general health condition, symptoms or degree of disease, gender, diet, excretion, components of other drug compositions used together simultaneously or separately to a subject. A person skilled in the art may easily determine and prescribe an effective dosage for the desired treatment. The pharmaceutical composition according to the present disclosure may be administered once a day or divided into several times. Therefore, these dosage does not limit the scope of the present disclosure. The dosage of the pharmaceutical composition may be 1 µg/kg/day to 1,000 mg/kg/day.

The subject may be a mammal, such as a human, cow, horse, pig, dog, sheep, goat, or cat. The subject may be an individual in need of hair loss treatment.

Another aspect is to provide an external skin preparation for improving a hair or scalp condition including hydrangenol represented by Formula 1 or a pharmaceutically acceptable salt thereof as an active ingredient.

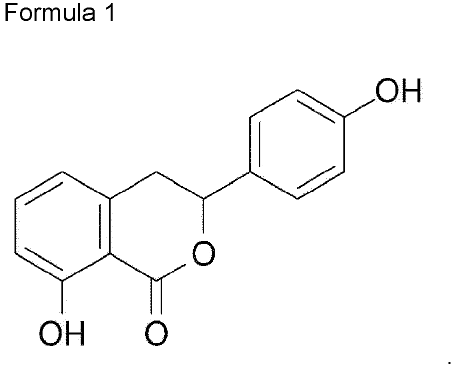

The external skin preparation may be a cream, gel, ointment, skin emulsifier, skin suspension, transdermal delivery patch, drug-including bandage, lotion, or a combination thereof. The external skin preparation may be appropriately mixed, according to need, with ingredients commonly used as an external skin preparation for use in cosmetics and medicines, such as aqueous ingredients, oil-based ingredients, powder ingredients, alcohols, humectants, thickeners, ultraviolet absorbers, whitening agents, preservatives, antioxidants, surfactants, fragrances, colorants, various skin nutrients, or a combination thereof. The external skin preparation may also be appropriately mixed with: metal sequestrants such as disodium edetate, trisodium edetate, sodium citrate, sodium polyphosphate, sodium metaphosphate, and gluconic acid; hot water extract of fruit, such as caffeine, tannin, belafamil, licorice extract, glablidin, and calin; drugs, such as various herbal medicines, tocopherol acetate, glytylitinic acid, and tranexamic acid and derivatives or salts thereof; vitamin C, magnesium ascorbate phosphate, ascorbate glucoside, and sugars, such as sugar, arbutin, kojic acid, glucose, fructose, and trehalose.

The skin includes all skin areas of the body, including the face, hands, arms, legs, feet, chest, stomach, back, buttocks, and the scalp.

The terms, methods, effects, etc. described for the present disclosure apply equally to each invention.

### Advantageous Effects of Disclosure

Regarding a composition including hydrangenol as an active ingredient according to an aspect, inhibits the activity of 5α-reductase, which is a hair loss mediating factor, promotes growth of dermal papilla cells, alleviates roughness of hair cuticles, and increases the tensile strength of hair. Accordingly, the composition can be effectively used to improve the condition of hair or scalp.

### Brief Description of Drawings

FIG. 1 shows an ESIMS (positive-ion mode) spectrum of hydrangenol.
FIG. 2 shows a ¹H-NMR spectrum of hydrangenol.
FIG. 3 shows a ¹³C-NMR spectrum of hydrangenol.
FIG. 4 shows a DEPT NMR spectrum of hydrangenol.
FIG. 5 shows a HSQC NMR spectrum of hydrangenol.
FIG. 6 shows a HMBC NMR spectrum of hydrangenol.
FIG. 7 is a graph comparing 5α-reductase enzyme inhibitory activity levels when treated with *Hydrangea serrata* extract and hydrangenol:
   FIG. 7A is a graph showing the 5α-reductase enzyme inhibitory activity level according to treatment with *Hydrangea serrata* extract; and FIG. 7B is a graph showing the 5α-reductase enzyme inhibitory activity level according to treatment with hydrangenol.
FIG. 8 is a graph confirming cytotoxicity in dermal papilla cells when treated with *Hydrangea serrata* extract and hydrangenol:
   FIG. 8A is a graph showing cytotoxicity levels in dermal papilla cells according to treatment with *Hydrangea serrata* extract; and FIG. 8B is a graph showing cytotoxicity levels in dermal papilla cells according to treatment with hydrangenol.
FIG. 9 is a graph confirming the cell proliferation effect in dermal papilla cells when treated with *Hydrangea serrata* extract and hydrangenol:
   FIG. 9A is a graph showing cell proliferation values in dermal papilla cells according to treatment with *Hydrangea serrata* extract; and FIG. 9B is a graph showing cell proliferation values in dermal papilla cells according to treatment with hydrangenol.
FIG. 10 is a graph of the tensile strength of the hair when treated with *Hydrangea serrata* extract and hydrangenol:
   FIG. 10A is a graph showing the tensile strength of the hair values according to treatment with *Hydrangea serrata* extract; and FIG. 10B is a graph showing the tensile strength of the hair values according to treatment with hydrangenol.
FIG. 11 is a graph of the roughness of hair cuticles when treated with *Hydrangea serrata* extract and hydrangenol:
   FIG. 11A is a graph showing hair cuticle roughness values according to treatment with *Hydrangea serrata* extract; and FIG. 11B is a graph showing hair cuticle roughness values according to treatment with hydrangenol.
FIG. 12 is a photograph of the roughness of hair cuticles when treated with *Hydrangea serrata* extract and hydrangenol:
   FIG. 12A is a photograph showing the roughness of hair cuticles according to treatment with *Hydrangea serrata* extract; and FIG. 12B is a photograph showing the roughness of hair cuticles according to treatment with hydrangenol.

### Mode of Disclosure

The present disclosure will be described in more detail through examples below. However, these examples are intended to illustrate one or more embodiments and the scope of the present disclosure is not limited to these examples.

### Example 1: Preparation of Hydrangea serrata extract

The *Hydrangea serrata* extract contained in the composition of the present disclosure was prepared by the following process.

First, *Hydrangea serrata* which grow naturally in Korea, was purchased and washed thoroughly with distilled water, and then completely dried in a cool place without sunlight until there was no change in weight. Afterwards, the completely dry product was pulverized using a homogenizer to obtain pulverized products. Then, 900 g of purified water was prepared, and then 30 g of the pulverized product was added. At this time, the mixing ratio of the pulverized product and purified water was set to be 1:30 in the weight ratio. Next, the purified water mixed with the pulverized product was placed in a constant temperature water bath and subjected to stirring and extraction at 100°C for 5 hours. The extracted sample was filtered through a cartridge filter (10 µm) and then concentrated under reduced pressure, and water-soluble powder was obtained through spray drying.

### Example 2: Preparation of Hydrangenol

Hydrangenol was prepared using the hydrangea extract powder obtained in Example 1.

First, gel filtration was performed on the extracted powder using Diaion HP-20. As a developing solvent, 2 L of a mixed solution of each of 30%, 50%, 70%, and 100% methanol and CH₂Cl₂-MeOH (1:1, v/v) was used for solvent fractionation, which was then divided into five small fractions (392-70EDia1 to 5). The small fraction 392-70EDia4 (357.4mg) was obtained by Sephadex LH-20, and divided into 7 small fractions (392-70EDia4a to 4g) using methanol as a developing solvent. From among the fractions, the 392-70EDia4d fraction was recrystallized in methanol to form Compound 1 which was in an amorphous form. Compound 1 was a pure hydrangenol single material.

Next, to identify the structure of Compound 1, ESIMS (positive-ion mode) spectrum, ¹H-NMR spectrum, ¹³C-NMR spectrum, DEPT NMR spectrum, 2D NMR spectrum, and HMBC spectrum were measured.

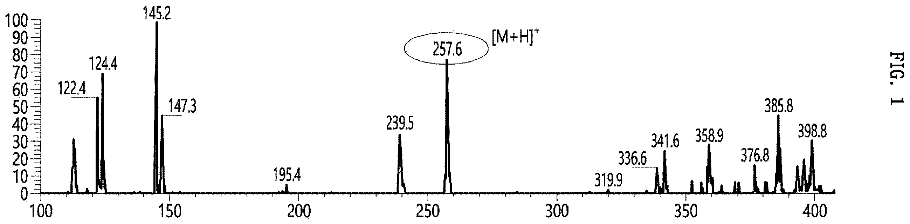

FIG. 1 shows an ESIMS (positive-ion mode) spectrum of hydrangenol.

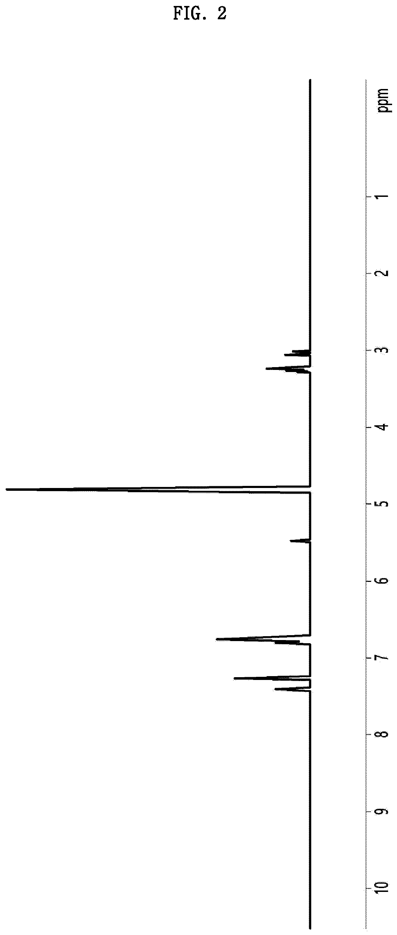

FIG. 2 shows a ¹H-NMR spectrum of hydrangenol.

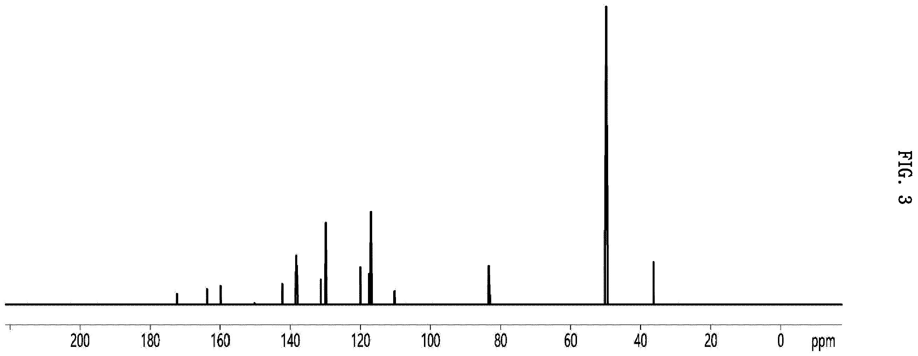

FIG. 3 shows a ¹³C-NMR spectrum of hydrangenol.

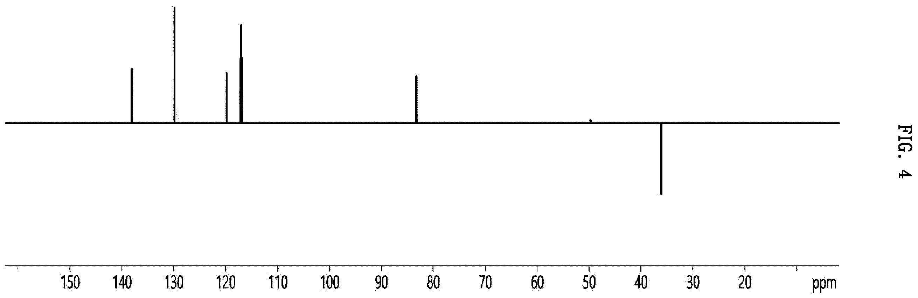

FIG. 4 shows a DEPT NMR spectrum of hydrangenol.

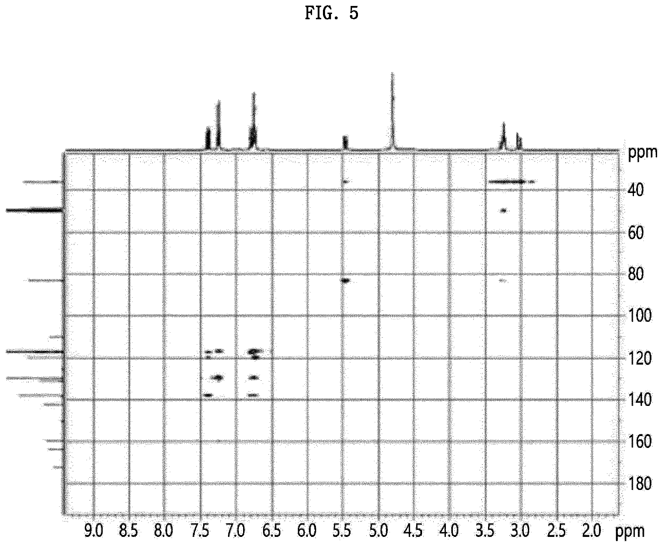

FIG. 5 shows a HSQC NMR spectrum of hydrangenol.

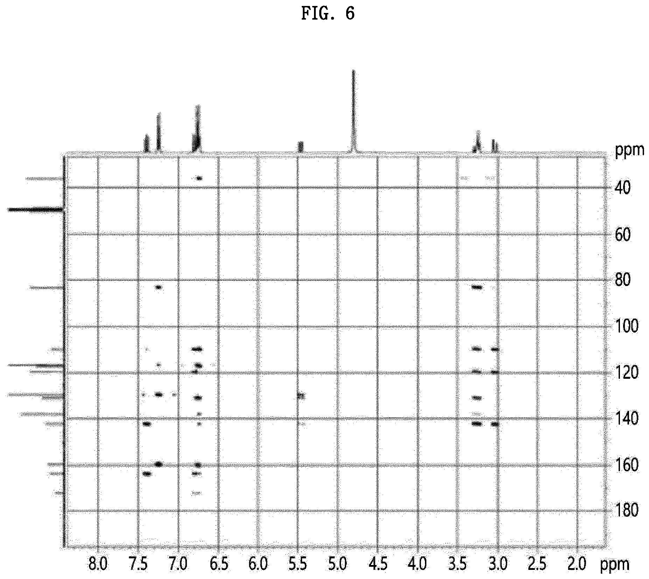

FIG. 6 shows a HMBC NMR spectrum of hydrangenol.

As a result, as shown in FIGS. 1 and 2, in the case of the ESIMS (positive-ion mode) spectrum, m/z = 257 [M+H]+; and in the case of the ¹H-NMR spectrum, the methine proton (H-3) at δH 5.50 and methylene proton (H-4) at δH3.30 and δH3.06 were, which appear at a high magnetic field, vicinal coupled to each other and from the chemical shift value, the proton was identified as being originating from the C ring. In addition, H-2', H-3', H-6', and H-5', resulting from the p-substituted benzene ring of the B-ring, were ortho coupled to each other and appeared as a doublet (J = 8.4 Hz), and the peaks of H-2' and H-6' and the peaks of H-3' and H-5' were ortho-coupled with each other and appeared as doublets, indicating a symmetrical structure around the hydroxy group. In addition, regarding 1,2,3-trisubstituted benzene of A-ring, hydrogens H-5 and H-7 were each coupled with hydrogen H-6, and hydrogens H-5 and H-7 were ortho coupled to show a doublet, and proton of H-6 was meta coupled with ortho to show doubles of doublets, and all were peaks corresponding to a single hydrogen.

In addition, as shown in FIGS. 3 and 4, it was confirmed that the ¹³C-NMR spectrum showed a total of 15 peaks, including the para-substituent. The quaternary carbon at δC 172 was a peak resulting from the carbonyl group, which was carbon of Compound 1, and δC 116.9 (C-3', C-5') and 129.6 (C-2', C-6') were the peaks originating from the para substituent of the aromatic ring, and the peaks at δC 36.1 and δC 83.1 were expected to originate from aliphatic carbon and oxygenated carbon, respectively. Additionally, in the case of the DEPT NMR spectrum, seven protonated carbons were identified, and the peak at δC 36.1 was found to be a methylene group originating from C-4.

In addition, according to FIGS. 5 and 6, the exact structure of Compound 1 could be confirmed by 2D NMR, and the exact positions of the peaks were identified from the HSQC spectrum to determine the position where the substituent was bound from HMBC. That is, the peak of δH 7.26 (2H, d,J = 8.4 Hz, H-2', H-6') was correlated with C-4 of δC 36.1, and the peaks of δH 3.06 and 3.30 originating from H-4 were correlated with the peaks of δC 83.1 (C-3), δC 119.8 (C-5), δC 110.0 (C-9), and δC 142.2 (C-10).

From these results and known references, Compound 1 was identified as hydrangenol (Yoshikawa M.,Matsuda H., Shimoda H., Shimada H., Harada E., Naitoh Y., Miki A., Yamahara J., Murakami N.Development of Bioactive Functions in Hydrangeae Dulcis Folium. V. On the Antiallergic and Antimicrobial Principles of Hydrangeae Dulcis Folium. (2). Thunberginols C, D, and E, Thunberginol G 3'-O-Glucoside, (-)-Hydrangenol 4'-O-Glucoside, and (+)-Hydrangenol 4'-O-Glucoside. Chem. Pharm. Bull.1996, 44: 1440-1447).

### Experimental Example 1: Confirmation of the effect of inhibiting 5α-reductase enzyme activity

The 5α-reductase enzyme inhibitory ability of each of *Hydrangea serrata* extract and hydrangenol obtained in Examples 1 and 2 was confirmed.

5α-reductase reduces testosterone to produce dihydrotestosterone (DHT). After reacting rat liver homogenate with testosterone, the ability to inhibit 5α-reductase enzyme activity was measured using a method of quantify testosterone.

First, a 7-week-old male SD rat was prepared and anesthetized using ethyl ether. After SD rats were laparotomised, the hepatic portal vein thereof was nicked, and cold STM buffer (270 mM sucrose, 10 mM Tris-HCl, pH7.5, 1 mM MgCl₂, G-biosciences, USA) was injected into the left ventricle using a syringe. The injection was continued until the STM buffer was perfused into the liver and the color of the liver changed from dark red to light pink. Afterwards, the liver was removed, transferred to a beaker, cut with scissors, and homogenized after adding STM buffer in an amount three times that of the liver. After centrifuging the homogenate at 10,000 xg for 10 minutes, the supernatant was homogenized once more to prepare an enzyme solution. The *Hydrangea serrata* extract of Example 1 and the hydrangenol of Example 2 were each added to phosphate buffer (pH 6.5), 500 µM testosterone, and 770 µM nicotinamide adenine dinucleotide phosphate (NADPH). Next, the prepared enzyme solution was added and reacted at 37°C for 30 minutes. To terminate the reaction, dichloromethane was added and centrifugation was performed at 2,000 rpm for 10 minutes. The dichloromethane layer was separated, concentrated, dissolved in 50% methanol, and quantified using high-performance chromatography. The 5α-reductase inhibition ability was calculated using the formula: 5α-reductase inhibition ability (%) = [1 - (area value of sample - area value of blank sample) / (area value of test group - area value of blank sample)] × 100 .

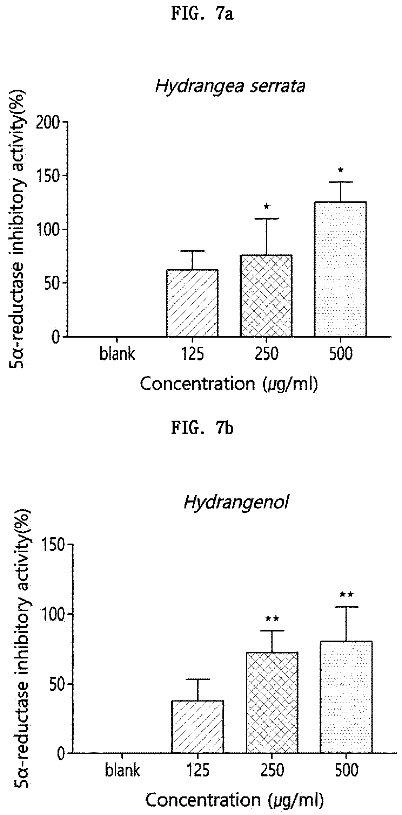

As shown in FIGS. 7A and 7B, *Hydrangea serrata* extract and hydrangenol were each treated at 125 µg/ml, 250 µg/ml, or 500 µg/ml, and were confirmed to exhibit 5α-reductase inhibitory activity in a concentration-dependent manner. These results indicate that *Hydrangea serrata* extract and hydrangenol are effective in suppressing or alleviating hair loss.

### Experimental Example 2: Confirmation of cytotoxicity

It was confirmed whether the *Hydrangea serrata* extract and hydrangenol obtained according to Examples 1 and 2 were cytotoxic to human follicle dermal papilla cells (HFDPC).

HFDPC cells were cultured in follicle dermal papilla cell growth medium including supplement mix (5% fetal calf serum, 0.5% bovine pituitary extract, 0.5 µg basic fibroblast growth factor, and 2.5 mg insulin) and 1% antibiotic antimycotic solution. HDFPC cell lines were seeded into a 96-well plate at a concentration of 1.0 × 10⁴ cells/well and incubated in a CO₂ incubator conditioned at 37°C, 95 % humidity, and 5 % CO₂ for 24 hours. Subsequently, the medium was replaced with medium without supplement mix and was treated with samples of each concentration and after 24 hours of incubation, cell viability was measured by MTT assay. The MTT assay is a method to measure the amount of formazan formed from MTT reduced by the mitochondria of living cells. More specifically, 100 µl of 0.5 mg/mL MTT solution was applied to each cell and incubated for 4 hours. Then, the solution was completely removed and the absorbance of the plate dissolved in DMSO was measured at 540 nm.

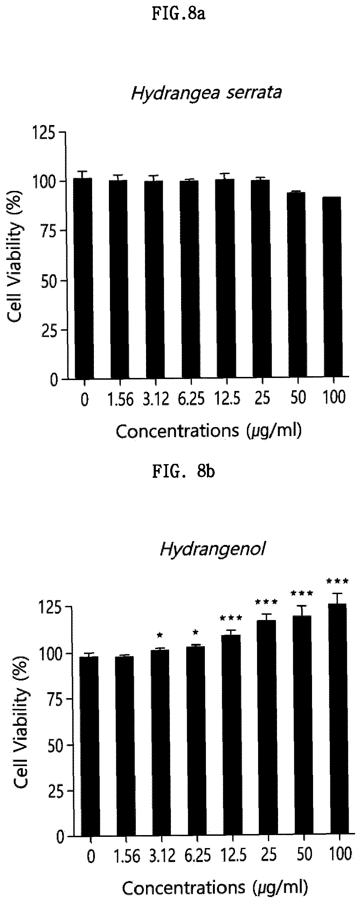

As shown in FIGS. 8A and 8B, *Hydrangea serrata* extract was treated at concentrations of 1.56 µg/ml, 3.12 µg/ml, 6.25 µg/ml, 12.5 µg/ml, 25 µg/ml, 50 µg/ml, and 100 µg/ml, and it was confirmed that there was no cytotoxicity at all concentrations. In addition, when treated with hydrangenol at concentrations of 1.56 µg/ml, 3.12 µg/ml, 6.25 µg/ml, 12.5 µg/ml, 25 µg/ml, 50 µg/ml, and 100 µg/ml, no cytotoxicity was shown at all concentrations. In particular, hydrangenol was confirmed to significantly increase cell growth at concentrations of 3.12 µg/ml to 100 µg/ml.

### Experimental Example 3: Confirmation of cell proliferation efficacy

Based on the results of the dermal papilla cell toxicity test confirmed above, the cell proliferation effect of the *Hydrangea serrata* extract and hydrangenol obtained in Examples 1 and 2 was confirmed. HFDPC cell lines were seeded into a 96-well plate at a concentration of 1.0 × 10³ cells/well and incubated in a CO₂ incubator conditioned at 37°C, 95 % humidity, and 5 % CO₂ for 24 hours. Subsequently, after treatment with samples at each concentration, cell proliferation was measured by MTT assay after 1, 3, and 6 days of incubation. More specifically, after treatment with samples of each concentration, the incubation was proceeded for 24, 72, and 144 hours, and then each cell was treated with 100 µl of 0.5 mg/ml MTT solution, and after 4 hours, the solution was completely removed and the plates dissolved in DMSO were measured at an absorbance of 540 µm.

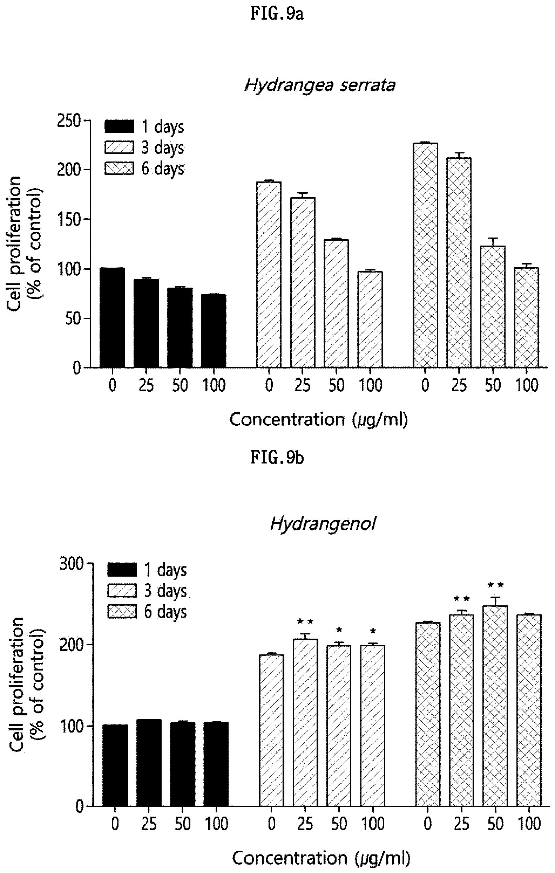

As shown in FIGS. 9A and 9B, when treated with hydrangenol for 3 and 6 days, cell growth was significantly increased at concentrations of 25 µg/ml and 50 µg/ml. The results of Experimental Example 2 and the present Experimental Example show that hydrangenol promotes hair cell growth.

### Experimental Example 4: Measurement of the tensile strength of the hair

The tensile strength of the hair was measured by treatment with each of *Hydrangea serrata* extract and hydrangenol obtained in Examples 1 and 2 above.

When the hair is pulled by applying force, the hair gradually stretches, becomes thinner, and eventually breaks. The force applied to the hair at this time is called tensile strength. The tensile strength of hair refers to the load at the time of cutting, and refers to the breaking strength at the moment the object is broken by external force when pulled. The measuring machine is a Universal Testing Machine (Instron3366, MA, USA), and the sampled hair was pulled at a speed of 20 mm/min. For each group, five threads, to which *Hydrangea serrata* extract or hydrangenol and the control group were applied, were used, and tensile strength measurements on hair were used as evaluation data. The unit of tensile strength was expressed as N (Newton), and a higher Newton value means higher tensile strength. Tensile strength was measured after repeating the cycle a total of 14 times, the cycle including leaving for 30 minutes after the application of a sample, then washing the hair with running water and drying the same naturally. *Hydrangea serrata* extract was dissolved at a concentration of 10 mg/ml in distilled water, and distilled water (negative control) was used as a control, and hydrangenol was dissolved at a concentration of 2 mg/ml in 1% DMSO, 1% Cremophor, and 98% distilled water, and a vehicle (solvent control group), to which 1% DMSO, 1% Cremophor, and 98% distilled water was added, was used as a control.

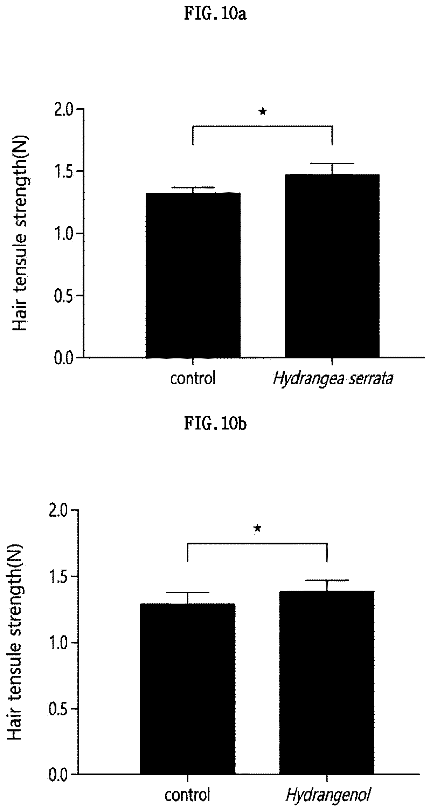

As shown in FIGS. 10A and 10B, the average values of 1.321 in the negative control group, 1.468 in the *Hydrangea serrata* extract, 1.288 in the solvent control group, and 1.373 in hydrangenol were confirmed, and there was a statistically significant difference between the negative control group and the *Hydrangea serrata* extract (p< 0.05): compared to the solvent control group, hydrangenol showed an increase in tensile strength. These results indicate that *Hydrangea serrata* extract and hydrangenol have the effect of strengthening or improving hair condition.

### Experimental Example 5: Measurement of roughness of hair cuticles

Roughness of hair cuticles was measured by treatment with each of *Hydrangea serrata* extract and hydrangenol obtained in Examples 1 and 2.

To measure the roughness of hair cuticles, the test area (surface of the hair) was photographed using a scanning electron microscope (SEM) and the roughness of the hair surface was analyzed using an image analysis program (ImageJ, NIH, USA). A SEM is a device operated such that when high-speed electrons are shoot at the surface of a sample, the electrons strike and interact with the surface of the sample, causing a material such as electrons to bounce out of the sample, and the material is then analyzed in three-dimensional form.

After the application of a sample on the hair, the hair was left untouched for 30 minutes, and then, washed with running water and dried naturally. This process was repeated a total of 14 times. *Hydrangea serrata* extract was dissolved in distilled water at a concentration of 10 mg/ml, and distilled water (negative control) was used as a control, and hydrangenol was dissolved at a concentration of 2 mg/ml in 1% DMSO, 1% Cremophor, and 98% distilled water, and a vehicle (solvent control group), to which 1% DMSO, 1% Cremophor, and 98% distilled water was added, was used as a control. Hair was collected, and each hair sample was cut into 4 mm long pieces and sampled. Then, platinum plating was performed to a thickness of 10 nm using an ion deposition machine, and then the coated sample was placed under a SEM and observed at 500x magnification in high vacuum. For the captured images, the Ra value, which is a variable representing the roughness of the surface, was calculated using surface roughness within the same area. A smaller Ra value indicates that the hair cuticle has improved.

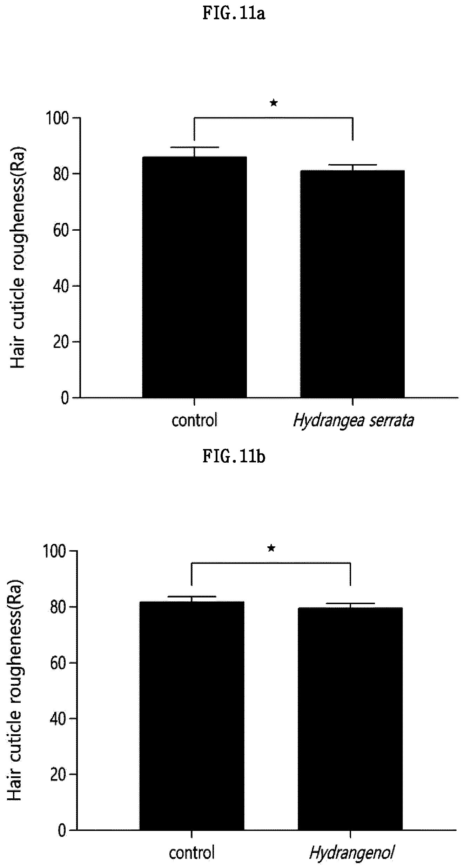

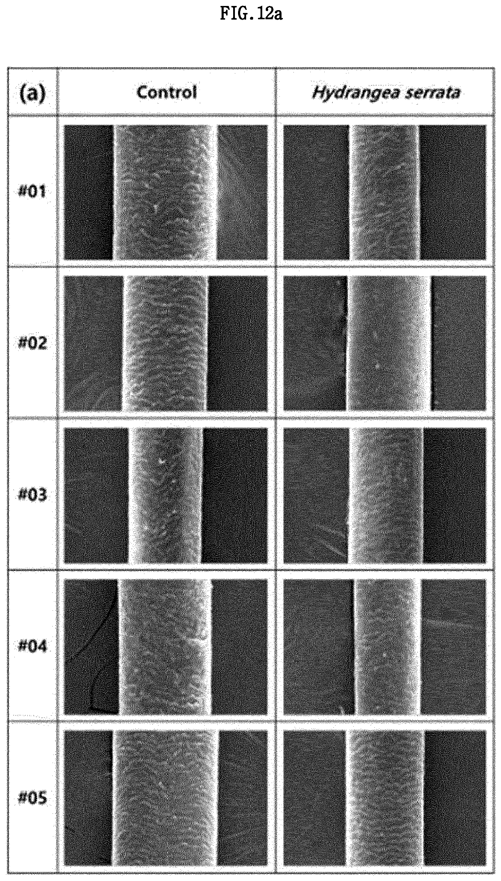

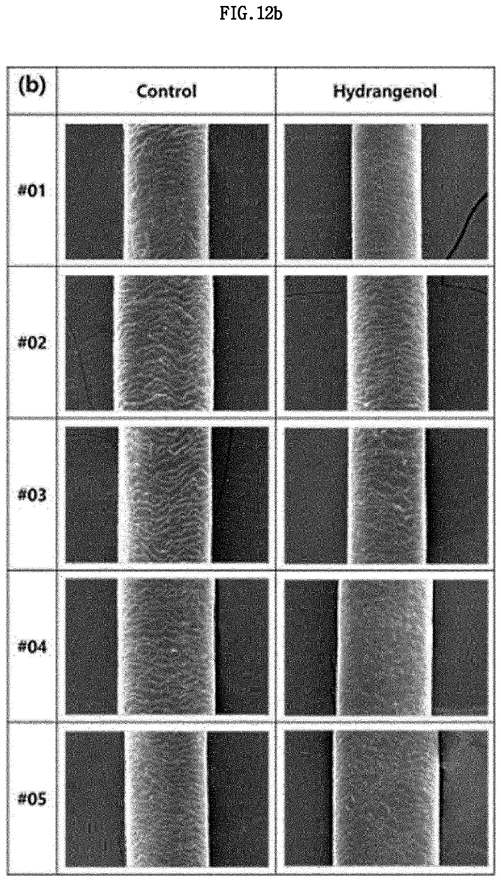

As shown in FIGS. 11A, 11B, 12A, and 12B, the average values of 86.189 in the negative control group, 81.168 in the *Hydrangea serrata* extract, 81.609 in the solvent control group, and 79.593 in hydrangenol were confirmed, and there was a statistically significant difference (p<0.05) between the negative control group and the *Hydrangea serrata* extract. When compared to the solvent control group, it was confirmed that the hair cuticle was significantly improved even when treated with hydrangenol. These results indicate that *Hydrangea serrata* extract and hydrangenol have the effect of strengthening or improving hair condition.

### Formulation Example 1: Preparation of tablets

With respect to Example 2, tablets were prepared by mixing and compressing the ingredients in Table 1 according to a tablet manufacturing method of the related art.

**Table 1**

| Raw material name | Unit weight (mg) |
|---|---|
| Example 2 | 10.0000 |
| Silicon dioxide | 15.3000 |
| Magnesium stearate | 10.8000 |
| Crystalline cellulose | 799.4951 |
| Hydroxypropylmethylcel lulose | 29.0700 |
| Carboxymethylcellulose Calcium | 27.0000 |
| Glycerin fatty acid ester | 0.6930 |
| Titanium dioxide | 1.4697 |
| Monascus red | 4.4082 |
| Powdered caramel coloring | 1.7640 |

### Formulation Example 2: Preparation of capsules

With respect to Example 2, soft capsules were prepared by mixing the ingredients in Table 2 below and filling the same into gelatin capsules according to a capsule manufacturing method of the related art.

**Table 2**

| Raw material name | Unit weight (mg) |
|---|---|
| Example 2 | 10 |
| Vitamin E | 2.25 |
| Vitamin C | 2.25 |
| Palm oil | 0.5 |
| Hdrogenated vegetable oil | 2 |
| Yellow lead | 1 |
| Lecithin | 2.25 |
| Soft capsule filling liquid | 427.75 |

### Formulation Example 3: Preparation of liquid formulation

With respect to Example 2, a liquid preparation was prepared by mixing the ingredients in Table 3 below and filling the same in a bottle or pouch according to a beverage preparation method suitable to preferences.

**[Table 3]**

| Raw material name | Unit weight (g) |
|---|---|
| Example 2 | 0.5050 |
| Xanthan gum | 0.0075 |
| Fructooligosacch aride solution | 0.7500 |
| Coconut flower essence powder | 1.0500 |
| Ssanghwa Concentrate | 1.5000 |
| Red ginseng scent | 0.0450 |
| Purified water | 11.1425 |

### Formulation Example 4: Preparation of jelly

With respect to Example 2, jelly was prepared by mixing the ingredients in Table 4 below and filling the same in a three-sided cloth according to a jelly manufacturing method suitable to preferences.

**[Table 4]**

| Raw material name | Unit weight (g) |
|---|---|
| Example 2 | 0.5000 |
| Food gel | 0.3600 |
| Carrageenan | 0.0600 |
| Calcium lactate | 0.1000 |
| Sodium citrate | 0.0600 |
| Complex golden extract | 0.0200 |
| Enzyme-processed stevia | 0.0440 |
| Fructooligosacch aride solution | 5.0000 |
| Red grape concentrate | 2.4000 |
| Purified water | 15.4560 |

These composition ratios are presented as a formulation example in which appropriate ingredients are mixed, but the mixing ratio and raw materials may be arbitrarily changed as needed.

The extract of the present disclosure or the compounds derived therefrom were stable under all formulation test conditions. Accordingly, no issues with formulation stability were encountered.

## Claims

1. A health functional food composition for improving a hair or scalp condition comprising hydrangenol represented by Formula 1 or a foodologically acceptable salt thereof as an active ingredient:

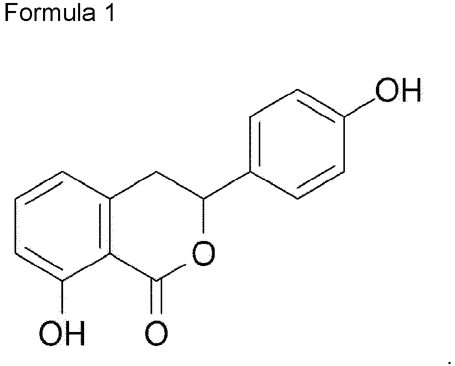

2. The health functional food composition for improving a hair or scalp condition of claim 1, wherein the hydrangenol is isolated from a *Hydrangea serrata* extract.

3. The health functional food composition for improving a hair or scalp condition of claim 1, wherein improving the hair or scalp condition includes: preventing or alleviating hair damage; promoting hair growth; or inhibiting, preventing, or alleviating hair loss.

4. The health functional food composition for improving a hair or scalp condition of claim 1, wherein the hydrangenol inhibits 5α-reductase and promotes proliferation of dermal papilla cells.

5. The health functional food composition for improving a hair or scalp condition of claim 1, wherein the hydrangenol increases the tensile strength of the hair and alleviates roughness of hair cuticles.

6. The health functional food composition for improving a hair or scalp condition of claim 1, wherein the health functional food has a formulation selected from the group consisting of powder, granules, tablets, capsules, pills, gels, jellies, suspensions, emulsions, syrups, tea bags, leached teas, and health drinks.

7. A cosmetic composition for improving a hair or scalp condition comprising hydrangenol represented by Formula 1 or a cosmetically acceptable salt thereof as an active ingredient:

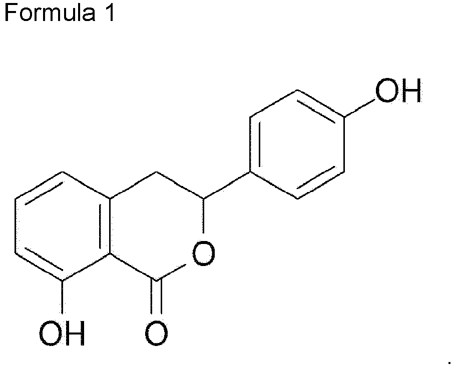

8. The cosmetic composition for improving a hair or scalp condition of claim 7, wherein the hydrangenol is isolated from a *Hydrangea serrata* extract.

9. The cosmetic composition for improving a hair or scalp condition of claim 7, wherein improving the hair or scalp condition includes: preventing or alleviating hair damage; promoting hair growth; or inhibiting, preventing, or alleviating hair loss.

10. A pharmaceutical composition for preventing or treating hair loss comprising hydrangenol represented by Formula 1 or a pharmaceutically acceptable salt thereof as an active ingredient:

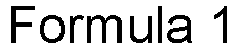

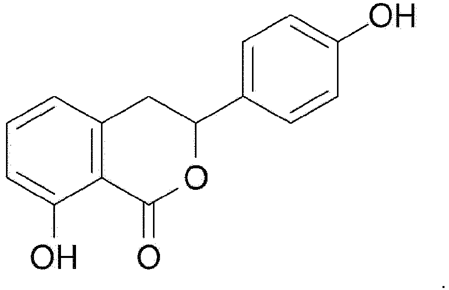

11. An external skin preparation for improving a hair or scalp condition comprising hydrangenol represented by Formula 1 or a pharmaceutically acceptable salt thereof as an active ingredient:

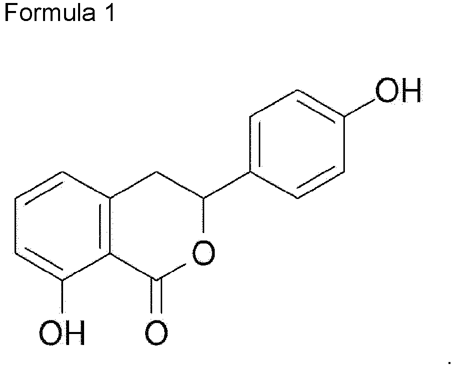

12. A method of preventing, alleviating, or treating hair loss, the method comprising administering an effective amount of hydrangenol represented by Formula 1 or a pharmaceutically acceptable salt thereof to a subject in need thereof.

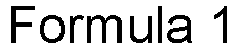

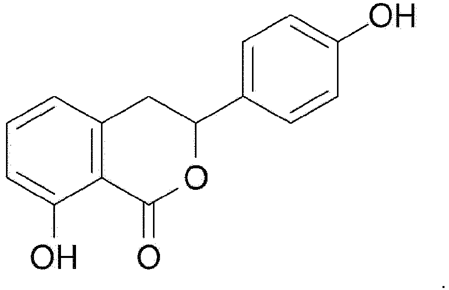

13. Use of hydrangenol represented by Formula 1 or a pharmaceutically acceptable salt thereof in the manufacture of a composition for improving a hair or scalp condition, or preventing, alleviating or treating hair loss.
